(19)

Europäisches
Patentamt
European
Patent Office
Office européen
des brevets

(11) **EP 4 411 751 A1**

(12) **EUROPEAN PATENT APPLICATION**
published in accordance with Art. 153(4) EPC

(43) Date of publication:
**07.08.2024 Bulletin 2024/32**

(21) Application number: **23866659.8**

(22) Date of filing: **30.06.2023**

(51) International Patent Classification (IPC):
***G16H 70/40*** (2018.01)

(52) Cooperative Patent Classification (CPC):
**Y02A 90/10**

(86) International application number:
**PCT/CN2023/105216**

(87) International publication number:
**WO 2024/131025 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2022 CN 202211667413**

(71) Applicants:
• **Nanqi Xiance (Nanjing) High Tech Co., Ltd.
Nanjing, Jiangsu 210000 (CN)**
• **Yu, Guo
Nanjing, Jiangsu 210000 (CN)**

(72) Inventors:
• **YU, Guo
Nanjing, Jiangsu 210000 (CN)**

• **YU, Yang
Nanjing, Jiangsu 210000 (CN)**
• **HAN, Luyao
Nanjing, Jiangsu 210000 (CN)**
• **LIU, Tianshuo
Nanjing, Jiangsu 210000 (CN)**
• **ZHANG, Zhilong
Nanjing, Jiangsu 210000 (CN)**
• **ZHAN, Dechuan
Nanjing, Jiangsu 210000 (CN)**
• **ZHANG, Qiang
Nanjing, Jiangsu 210000 (CN)**

(74) Representative: **Weickmann & Weickmann
PartmbB
Postfach 860 820
81635 München (DE)**

(54) **DATA PROCESSING METHOD AND APPARATUS, ELECTRONIC DEVICE, AND STORAGE
MEDIUM**

(57)    Provided are a data processing method and apparatus, an electronic device, and a storage medium. The method includes the following: The physiological feature data of a target object under at least one physiological indicator is acquired (S110); a data processing type corresponding to the physiological feature data is determined (S120), and a target network model corresponding to the data processing type is invoked; and the physiological feature data is processed (S130) based on the target network model to obtain target physiological feature data.

```
                                                          ┌─ S110
┌────────────────────────────────────────────────────┐
│ Acquire physiological feature data of a target      │
│ object under at least one physiological indicator   │
└────────────────────────────────────────────────────┘
                          │
                          ▼                               ┌─ S120
┌────────────────────────────────────────────────────┐
│ Determine a data processing type corresponding to   │
│ the physiological feature data, and invoke a target  │
│ network model corresponding to the data             │
│ processing type                                     │
└────────────────────────────────────────────────────┘
                          │
                          ▼                               ┌─ S130
┌────────────────────────────────────────────────────┐
│ Process the physiological feature data based on the │
│ target network model to obtain target physiological  │
│ feature data                                        │
└────────────────────────────────────────────────────┘
```

**FIG. 1**

Processed by Luminess, 75001 PARIS (FR)

**Description**

The present application claims priority to Chinese Patent Application No. 202211667413.6 filed with the China National Intellectual Property Administration (CNIPA) on Dec. 23, 2022, the disclosure of which is incorporated herein by reference in its entirety.

TECHNICAL FIELD

The present application relates to the field of data processing technology, for example, a data processing method and apparatus, an electronic device, and a storage medium.

BACKGROUND

In the stage of drug clinical development, it is often necessary to evaluate the pharmacokinetic behaviors of a drug. For example, a pharmacokinetic model may be used to evaluate the difference in pharmacokinetic behaviors among individuals caused by physiological features of the group of individuals.

When the differences between individuals are evaluated, a fixed equation between group measurement data and intrinsic physiological features is usually established according to the reported prior knowledge, and a virtual individual group is established according to the designated group measurement data. However, because of the differences in growth curves of individual physiological features, the mechanism correlation between anthropometric data and intrinsic physiological features distinguishes not only patterns between children and adults but also patterns among different physiological features of individuals, which leads to the generated distribution far different from the distribution of physiological features of each group in real life, and physiological feature data cannot be accurately processed.

SUMMARY

The present application provides a data processing method and apparatus, an electronic device, and a storage medium to solve a problem that physiological feature data samples are insufficient when a medicinal property is evaluated based on physiological feature data or that simulated physiological feature data does not conform to the distribution of real physiological feature data in the simulation of physiological feature data.

In a first aspect, an embodiment of the present application provides a data processing method. The method includes the steps below.

Physiological feature data of a target object under at least one physiological indicator is acquired.

A data processing type corresponding to the physiological feature data is determined, and a target network model corresponding to the data processing type is invoked. The data processing type includes a data generalization type or a data prediction type.

The physiological feature data is processed based on the target network model to obtain target physiological feature data.

In a second aspect, an embodiment of the present application provides a data processing apparatus. The apparatus includes a data acquisition module, a model invocation module, and a data determination module.

The data acquisition module is configured to acquire physiological feature data of a target object under at least one physiological indicator.

The model invocation module is configured to determine a data processing type corresponding to the physiological feature data and invoke a target network model corresponding to the data processing type, where the data processing type comprises a data generalization type or a data prediction type.

The data determination module is configured to process the physiological feature data based on the target network model to obtain target physiological feature data.

In a third aspect, an embodiment of the present application further provides an electronic device. The electronic device includes at least one processor and a memory communicatively connected to the at least one processor.

The memory stores a computer program executable by the at least one processor, and the computer program is executed by the at least one processor to cause the at least one processor to perform the data processing method according to any embodiment of the present application.

In a fourth aspect, an embodiment of the present application further provides a computer-readable storage medium storing computer instructions configured to, when executed, cause a processor to perform the data processing method according to any embodiment of the present application.

BRIEF DESCRIPTION OF DRAWINGS

To illustrate the technical schemes in embodiments of the present application more clearly, the accompanying drawings used in the embodiments are briefly described below.

FIG. 1 is a flowchart of a data processing method according to embodiment one of the present application.

FIG. 2 is a flowchart of a data processing method according to embodiment two of the present application.

FIG. 3 is a flowchart of determining a target network model according to embodiment two of the present application.

FIG. 4 is a structural diagram of a data processing apparatus according to embodiment three of the present application.

FIG. 5 is a structural diagram of an electronic device for implementing a data processing method according to an embodiment of the present application.

DETAILED DESCRIPTION

Technical schemes in embodiments of the present application will be described in conjunction with drawings in embodiments of the present application.
The terms "first", "second" and the like in the description, claims and preceding drawings of the present application are used for distinguishing between similar objects and are not necessarily used for describing a particular order or sequence. It is to be understood that the data used in this manner is interchangeable where appropriate so that embodiments of the present application described herein can also be implemented in a sequence not illustrated or described herein.

Embodiment one

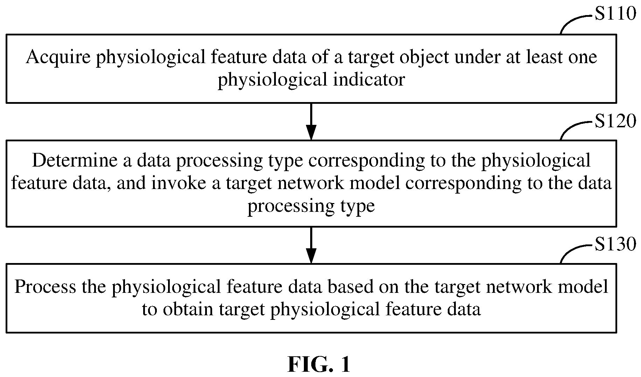

FIG. 1 is a flowchart of a data processing method according to embodiment one of the present application. This embodiment is applicable to the case where a large number of physiological feature data samples are obtained by simulating a small amount of user physiological feature data on the premise of conforming to the distribution law of real physiological feature data. This embodiment may be performed by a data processing apparatus. The data processing apparatus may be implemented by software and/or hardware and configured in a computing device that may perform a data processing method.
As shown in FIG. 1, the method includes the steps below.
In S 110, physiological feature data of a target object under at least one physiological indicator is acquired.
In this technical scheme, the target object is referred to as an experimental user whose physiological feature data is to be collected. For example, the target object may be an adult, a child, or an old person. The gender of the target object is not limited. A physiological indicator may be understood as an indicator for representing a physiological feature of the target object, for example, height, weight, blood glucose, blood pressure, pulse, vital capacity, temperature, oxygen saturation, or the weight and size of body tissue of the target user. The physiological indicator in this technical scheme may be used for representing multiple indicators of user physiological features and is not limited to the physiological indicator mentioned in this technical scheme. The physiological feature data may be understood as the measurement data corresponding to the at least one physiological indicator of the target object.
In practical applications, for example, in the stage of drug clinical development, a large amount of user physiological feature data usually need to be acquired to determine the effectiveness and safety of a drug so as to quantitatively study the absorption, distribution, metabolism and excretion of the drug in organisms during drug research and development. It is to be understood that the larger the quantity of physiological feature data of group users, the more accurate the experimental result of the drug. Therefore, a large amount of user physiological feature data needs to be acquired in the stage of medical clinical research. However, due to an insufficient quantity of collected real user physiological feature data, it is usually necessary to simulate user physiological feature data to obtain more user physiological feature data. It is to be understood that physiological feature data of the same user under multiple physiological indicators always have a certain association relationship. To make the simulated physiological feature data closer to the real physiological feature data, it is necessary to analyze the association relationship between the physiological feature data under multiple physiological indicators so that more real physiological feature data is obtained based on the association relationship between the physiological feature data in the simulation process.
On this basis, in practical applications, data generalization or data prediction may be performed based on the acquired physiological feature data under at least one physiological indicator to obtain more physiological feature sample data. When the physiological feature data of the target object under the at least one physiological indicator is acquired, the physiological feature data of the target object may be collected in reality. Alternatively, one or more users are selected as target objects from a public data set or a medical information system, and physiological feature data of the target objects under at least one physiological indicator is acquired. The public data set includes, but is not limited to, a data set based on a medical institution inspection information system, a data set based on a medical image transmission system, and other data sets including group physiological feature data. In this technical scheme, the data set including physiological feature data is not limited.

In S 120, a data processing type corresponding to the physiological feature data is determined, and a target network model corresponding to the data processing type is invoked.

The data processing type includes a data generalization type or a data prediction type. The target network model may be understood as a model for data generalization or data prediction based on the physiological feature data. Exemplary, the target network model may be a generation model, for example, a generative adversarial network model, a variational autoencoder model, a diffusion model, or a flow-based generation model.

The target object is taken for example. The physiological feature data of the target object under the at least one physiological indicator always have a certain association relationship. Therefore, the determination of the association relationship between the physiological feature data of the target object is of great importance for data generalization or data prediction based on the physiological feature data of the target object. In this technical scheme, the target network model may analyze the physiological feature data and determine the association relationship between the physiological feature data. The method for determining the target network model is described in subsequent embodiments.

To acquire physiological feature data of more users under the at least one physiological indicator, data simulation may be performed according to the physiological feature data of known users based on the target network model to obtain a larger amount of more real physiological feature data. The data processing type corresponding to the physiological feature data may be determined according to actual data processing requirements. For example, data generalization needs to be processed based on the physiological feature data of the target object. Alternatively, data prediction needs to be processed based on the physiological feature data of the target object. After the data processing type corresponding to the physiological feature data is determined, the target network model is invoked to process the physiological feature data so that the target physiological feature data corresponding to the target object is obtained.

The step in which the data processing type corresponding to the physiological feature data is determined and the target network model corresponding to the data processing type is invoked includes the following: A data processing instruction is received, and a data processing manner in the data processing instruction is acquired; the corresponding data processing type is determined based on the data processing manner and the physiological feature data, and the target network model corresponding to the data processing type is invoked.

The data processing manner includes a data generalization manner or a data prediction manner.

In this technical scheme, different data processing requirements may correspond to different data processing manners. For example, the data generalization manner performed on data and the data prediction manner performed on data may be included.

In S130, the physiological feature data is processed based on the target network model to obtain target physiological feature data.

The target physiological feature data may be understood as the physiological feature data obtained after data prediction or data generalization is performed on the physiological feature data based on the target network model.

The physiological feature data of the target object is input into the target network model. Moreover, data prediction or data generalization is performed on the physiological feature data based on the target network model to obtain the target physiological feature data.

In an embodiment, the data processing manner is the data generalization manner. The step in which the physiological feature data is processed based on the target network model to obtain the target physiological feature data includes the following: A physiological feature curve corresponding to the physiological feature data is determined based on the target network model; a data floating range corresponding to the physiological feature curve is determined to obtain a preset number of physiological feature generalization curves from the data floating range; and at least one group of target physiological feature data corresponding to the physiological feature data is obtained based on the physiological feature generalization curves.

A physiological feature curve may be understood as a curve generated based on the physiological feature data. In this technical scheme, before the physiological feature data of the target object is input into the target network model, normalization needs to be performed on the physiological feature data. That is, the physiological feature data under at least one physiological indicator is processed to be in a range of 0 to 1 so that data analysis is performed on the normalized physiological feature data by using the target network model. The corresponding physiological feature curve is obtained according to the physiological feature data floating between 0 and 1. A physiological feature generalization curve may be understood as a physiological feature curve determined based on the physiological feature curve corresponding to the target object, and the physiological feature generalization curve is similar to the physiological feature curve. That is, the physiological feature generalization curve is obtained by simulating the physiological feature curve of the target object.

The target network model may obtain the association relationship between the physiological feature data under multiple physiological indicators by analyzing and processing the physiological feature data of the target object. On this basis, when data generalization is required, the physiological feature data of the target object under the at least one physiological indicator may be input into the target network model. Moreover, the data generalization number and the data floating range may be preset. For example, the height and weight are taken for example. The data generalization number is preset to 100. The data floating range for the height of the target object is ± 5 cm and the data floating range for the weight of the target object is ± 10 kg. On this basis, the physiological feature data of the target object under the at least one physiological indicator is input into the target network model. The association relationship between the physiological feature data may be obtained through the analysis on the physiological feature data of the target object. The corresponding physiological feature curve is generated based on the association relationship. The corresponding physiological feature generalization curve may be generated according to the preset data generalization number and data floating range, and each physiological feature generalization curve corresponds to a group of physiological feature data. Therefore, the corresponding target physiological feature data may be obtained based on the physiological feature generalization curve.

In this manner, the target physiological feature data is obtained based on the generalization of the physiological feature data of the target object, and the manner may be used to generate physiological feature data within a specified physiological feature range in batches. That is, when a large amount of physiological feature data is required, a large amount of target physiological feature data under a corresponding physiological indicator may be obtained according to the physiological feature data of the target object under the at least one physiological indicator having a small data amount. Moreover, the association relationship between the target physiological feature data is similar to the association relationship between the input physiological feature data, which conforms to the association relationship between the real physiological feature data. That is, a large amount of physiological feature data may be acquired in batches on the premise of guaranteeing the authenticity of physiological feature data to a certain extent.

In an embodiment, the data processing manner is the data prediction manner. The step in which the physiological feature data is processed based on the target network model to obtain the target physiological feature data includes the following: At least one to-be-determined physiological feature curve matching the physiological feature data is determined based on the target network model; a target physiological feature curve is determined from the at least one to-be-determined physiological feature curve according to basic attribute information corresponding to the target object; and the target physiological feature data corresponding to the target object is determined based on the target physiological feature curve.

It is to be understood that the corresponding physiological feature curve may be obtained based on the physiological feature data of the target object under the at least one physiological indicator. A to-be-determined physiological feature curve may be understood as a physiological feature curve having a variation law similar to the variation law of the physiological feature curve of the target object. The basic attribute information may include basic information such as the gender and age of the target object. The target physiological feature curve is a curve having the highest similarity with the physiological feature curve of the target object in the at least one to-be-determined physiological feature curve.

Exemplarily, in the case where the data processing manner is the data prediction manner, the physiological feature data of the target object is input into the target network model on the premise that the physiological feature data of the target object under one or more physiological indicators is known so that at least one to-be-determined physiological feature curve relatively close to the input physiological feature data is determined based on the target network model and the target physiological feature curve is determined from the at least one to-be-determined physiological feature curve according to the basic attribute information of the target object. The target physiological feature data corresponding to the target object may be obtained based on the target physiological feature curve. For example, the height and weight of the target object are known.

The physiological feature data corresponding to physiological indicators including the heart rate, pulse, and blood pressure of the target object may be determined according to the target network model.

In an embodiment, the at least one physiological indicator in this technical scheme includes at least one of height, weight, temperature, blood pressure, electrocardiogram information, or biological tissue information. The biological tissue information may be information such as the weight and size of an organ or tissue in an organism.

The physiological feature data corresponding to each physiological indicator in this technical scheme is acquired from a compliant and legal channel.

For the technical scheme in the embodiment of the present application, the physiological feature data of the target object under at least one physiological indicator is acquired. The physiological feature data of the target object is collected; alternatively, the physiological feature data of the target object under at least one physiological indicator is acquired from a pre-established or existing public data set. The data processing type corresponding to the physiological feature data is determined according to data processing requirements in practical applications, and the target network model corresponding to the data processing type is invoked so that the association relationship between the physiological feature data under multiple physiological indicators is analyzed based on the target network model to obtain the target physiological feature data corresponding to the data processing type. The physiological feature data is processed based on the target network model to obtain the target physiological feature data. When data generalization is processed, the physiological feature curve corresponding to the physiological feature data of the target object is determined based on the target network model. Moreover, data generalization is processed based on the physiological feature curve according to the preset data generalization number and data generalization range to obtain at least one target physiological feature curve. When data prediction is processed, the physiological feature data of the target object under other physiological indicators may be obtained through the physiological feature data of the target object under at least one physiological indicator and the basic attribute information of the target object so as to perform data prediction. In this manner, it is avoided the case where physiological feature data samples are insufficient when a medicinal property is evaluated based on the physiological feature data or the case where simulated physiological feature data does not conform to the distribution of real physiological feature data in the simulation of physiological feature data, and the effect of obtaining sufficient physiological feature data samples can be achieved by performing data generalization based on a small amount of physiological feature data on the premise of conforming to the distribution law of real physiological feature data.

Embodiment two

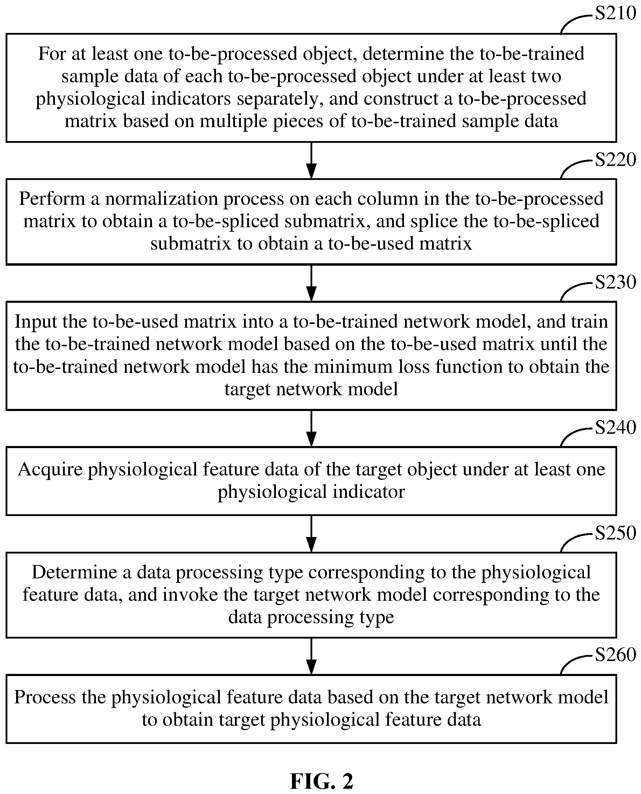

FIG. 2 is a flowchart of a data processing method according to embodiment two of the present application. In an embodiment, before the physiological feature data of the target object under at least one physiological indicator is acquired, the target network model needs to be pre-constructed to process the acquired physiological feature data based on the target network model.

In this technical scheme, the target network model may be a generation model, for example, a generative adversarial network model, a variational autoencoder model, a diffusion model, or a flow-based generation model. When the model is trained, any generation model may be selected to serve as a to-be-trained network model for training to obtain the target network model.

To more clearly introduce the construction method of the target network model, an example in which the target network model is a diffusion model is taken for introduction in this embodiment.

As shown in FIG. 2, the method includes the steps below.

In S210, for at least one to-be-processed object, to-be-trained sample data of each to-be-processed object under at least two physiological indicators is determined separately, and a to-be-processed matrix is constructed based on multiple pieces of to-be-trained sample data.

In this technical scheme, before the physiological feature data of the target object is processed based on the target network model, the to-be-trained network model needs to be trained first to obtain the target network model. When the to-be-trained network model is trained, a large number of physiological feature data of sample objects under at least one physiological indicator are used. A to-be-processed object is a sample object in the training process of the to-be-trained network model. The to-be-trained sample data is physiological feature data of a to-be-processed object under at least two physiological indicators. The to-be-processed matrix may be understood as a matrix obtained based on the combination of the to-be-trained sample data of each to-be-processed object. A row element of the to-be-processed matrix corresponds to the to-be-trained sample data of each to-be-processed object under at least two physiological indicators. Each column element corresponds to a different physiological indicator.

A large amount of sample data needs to be acquired to train the to-be-trained network model so that the target network model with a more accurate data generalization or prediction result is obtained. The to-be-trained sample data of at least one to-be-processed object under at least two physiological indicators is acquired to train the target network model based on the to-be-trained sample data.

For each to-be-processed object, the reason for acquiring the physiological feature data of each to-be-processed object under the at least two physiological indicators lies in that physiological feature data of the same to-be-processed object under different physiological indicators always have a certain association relationship. If only the physiological feature data of the to-be-processed object under one corresponding physiological indicator is acquired, the association relationship between physiological feature data under different physiological indicators cannot be acquired in the training process of the to-be-trained network model. On this basis, for each to-be-processed object, the physiological feature data under the at least two physiological indicators needs to be acquired to train the to-be-trained network model to obtain the target network model. Moreover, data generalization or data prediction is performed on the physiological feature data of the target object based on the target network model.

In S220, normalization is performed on each column in the to-be-processed matrix to obtain a to-be-spliced submatrix, and splicing is performed on the to-be-spliced submatrix to obtain a to-be-used matrix.

In the to-be-processed matrix, to-be-trained sample data corresponding to each column of elements is physiological feature data under the same physiological indicator. Normalization is performed on the to-be-trained sample data in each column to obtain the normalization data under a corresponding physiological indicator. A set formed by the normalized data of each column is taken as the to-be-spliced submatrix, and splicing is performed to obtain the to-be-used matrix. In other words, each element in the to-be-used matrix corresponds to data after the to-be-trained sample data is normalized.

When the to-be-trained sample data under each physiological indicator is normalized, the maximum value and minimum value in each column of data need to be recorded and are used as the reference values when the target network model restores the normalized data. Exemplarily, height is taken for example, the maximum value is 1.9 m, and the minimum value is 0.8 m. After normalization, 1 in the to-be-used matrix corresponds to the height of 1.9 m, and 0 in the to-be-used matrix corresponds to the height of 0.8 m. Data between 0 and 1 in the to-be-used matrix are between 0.8 m and 1.9 m. On this basis, after data generalization or data prediction is performed on the target network model, the obtained data is the normalized data between 0 and 1. Then the normalized data may be restored to the corresponding physiological feature data according to the maximum value and the minimum value corresponding to the physiological feature data under each physiological indicator.

In S230, the to-be-used matrix is input into the to-be-trained network model, and the to-be-trained network model is trained based on the to-be-used matrix until the to-be-trained network model has the minimum loss function to obtain the target network model.

The to-be-trained network model includes any one of a generative adversarial network model, a variational autoencoder model, a diffusion model, or a flow-based generation model.

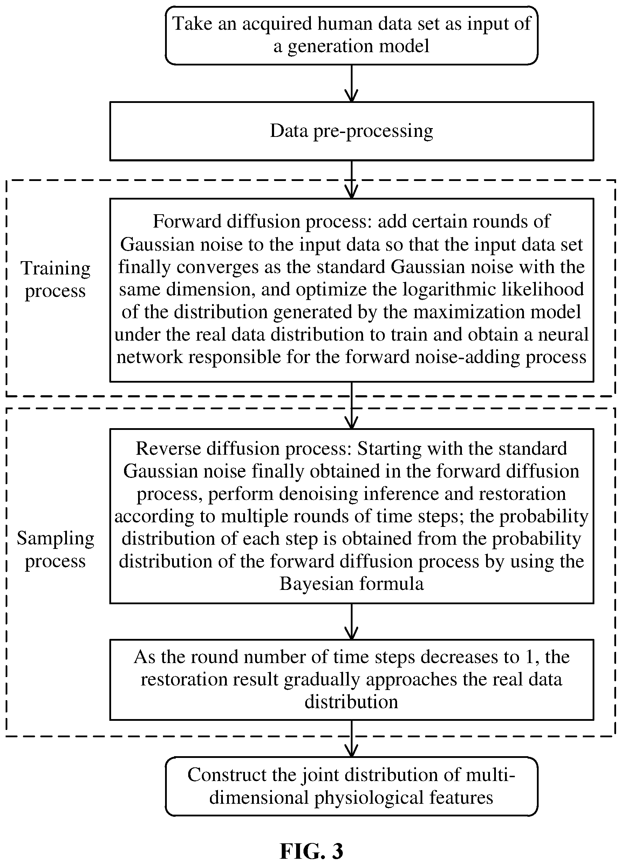

An example in which the to-be-trained network model is a diffusion model is taken for example. As shown in FIG. 3, in a forward diffusion process, certain rounds of Gaussian noise are added to the input data. Each round of Gaussian noise uses a different mean and variance, and the variance of the Gaussian distribution increases with time. Starting from any distribution, sampling is performed along a Markov chain. The final stationary distribution is the standard Gaussian distribution. In this process, the input data set, that is, the to-be-used matrix finally converges as the standard Gaussian noise with the same dimension, thereby completing the reconstruction of the input to-be-used matrix. Based on the characteristics of the diffusion model, the noise-adding result $x_t$ at any moment may be described as a linear combination of the initial value $x_0$ and the random added noise $z_t$, and the formula is as follows:

$$x_t = \sqrt{\overline{\alpha_t}}x_0 + \sqrt{1 - \overline{\alpha_t}}\overline{z}_t.$$

xt denotes the noise-adding result after noise is added to the to-be-used matrix at moment t. $x_0$ denotes the initial value in the to-be-used matrix. $\overline{z}_t$ denotes noise sampled from the standard Gaussian distribution. $\overline{\alpha}_t$ denotes the factorial of $\alpha$ from moment 0 to moment t. $\alpha$ denotes the hyperparameter of the variance of Gaussian noise.

In the training process of the diffusion model, an optimization target is the logarithmic likelihood of the distribution generated by a maximization model of data distribution. A loss function corresponding to the optimization target is as follows:

$$\mathcal{L} = \mathbb{E}_{q(x_0)}[-\log p_\theta(x_0)].$$

$q(x_0)$ denotes the real distribution of data input into the to-be-used matrix. $p_\theta(x_0)$ denotes the data distribution generated by the to-be-trained network model. $\theta$ denotes the parameter of a depth neural network. $\mathbb{E}_{q(x_0)}$ denotes a value of the expectation for the probability density function $q(x_0)$ of $x_0$. $\mathcal{L}$ denotes the loss function. The optimization target is simplified through variational inference. The target loss of the diffusion model can be minimized by minimizing the variational upper limit of the negative logarithmic likelihood. The simplified loss function is as follows:

$$\mathbb{E}_{x_0,\overline{z}_t}\left[\| \overline{z}_t - z_\theta\left(\sqrt{\overline{\alpha_t}}x_0 + \sqrt{1 - \overline{\alpha_t}}\overline{z}_t, t\right) \|^2\right].$$

$\mathbb{E}_{x_0,\overline{z}_t}$ denotes the value of the expectation when $x_0$ obeys the distribution of the data set and $\overline{z}_t$ obeys the standard Gaussian distribution. t denotes the number of rounds sampled from the total round number according to uniform distribution. $x_0$ denotes the initial data in the to-be-used matrix. $\overline{z}_t$ denotes the noise sampled from the standard Gaussian distribution. $z_\theta$ denotes the neural network for the training process of the diffusion model. $\overline{\alpha}_t$ denotes the factorial of $\alpha$ from moment 0 to moment t. $\alpha$ denotes the hyperparameter of the variance of Gaussian noise. $\|\cdot\|^2$ denotes a 2-norm. On this basis, the to-be-trained network model is trained to obtain the neural network responsible for the forward noise-adding process.

The sampling process of the diffusion model is taken for example. Denoising inference is performed in the reverse diffusion process. The process is also performed according to multiple rounds of time steps. The probability distribution of each step is obtained from the probability distribution of the forward diffusion process by using the Bayesian formula. Starting with the standard Gaussian noise finally obtained in the forward diffusion process, the restoration result and time step information of the previous round are used as the input of the neural network obtained by training in each round. Further, the restoration result of the current round is obtained by using the re-parameterization method. As the time step decreases from the total round number T to the total round number 1, the restoration result gradually approaches the real data distribution. Finally, the restoration of the joint distribution of multi-dimensional physiological features in the input human data set is completed.

The iteration formula for each time step in the iteration process is as below.

$$X_{t-1} = \frac{1}{\sqrt{\overline{\alpha}_t}} \left( X_t - \frac{1 - \alpha_t}{\sqrt{1 - \overline{\alpha}_t}} \epsilon_\theta(X_t, t) \right) + \sigma_t Z$$

$X_{t-1}$ denotes a result obtained by restoring Gaussian noise to moment t - 1 in the reverse process. $X_t$ denotes a result obtained by restoring Gaussian noise to moment t in the reverse process. $\overline{\alpha}_t$ denotes the factorial from moment 0 to moment t. a denotes the hyperparameter of the variance of Gaussian noise. $\varepsilon_\theta$ denotes the neural network obtained through the training process. Z denotes the noise sampled from the Gaussian distribution and has the same dimension as $X_t$. Because the variance $\sigma_t$ is difficult to solve, (1 - $\alpha$) is directly used instead in the implementation process.

Based on the training of the preceding to-be-trained network model, the target network model may be obtained when the loss function of the to-be-trained network model is minimal so that data processing is performed on the physiological feature data of the target object under at least one physiological indicator based on the target network model.

In S240, physiological feature data of the target object under the at least one physiological indicator is acquired.

In S250, a data processing type corresponding to the physiological feature data is determined, and the target network model corresponding to the data processing type is invoked.

In S260, the physiological feature data is processed based on the target network model to obtain target physiological feature data.

For the technical scheme in this embodiment, for at least one to-be-processed object, the to-be-trained sample data of each to-be-processed object under at least two physiological indicators is determined separately, and the to-be-processed matrix is constructed based on the to-be-trained sample data. Normalization is performed on each column in the to-be-processed matrix to obtain the corresponding to-be-spliced submatrix, and splicing is performed on the to-be-spliced submatrix to obtain the to-be-used matrix. The to-be-used matrix includes normalized data corresponding to the multiple pieces of to-be-trained sample data. The to-be-used matrix is input into the to-be-trained network model, and the to-be-trained network model is trained based on the to-be-used matrix to obtain the target network model when the loss function of the to-be-trained network model is minimal. In the process of training the to-be-trained network model, the neural network responsible for the forward noise-adding process is obtained by adding noise to the to-be-used matrix. The neural network is denoised to implement reconstruction of the to-be-used matrix. Moreover, the target network model is obtained in the case of the minimum loss function of the to-be-trained network model. According to the maximum value and minimum value of elements in each column in the to-be-used matrix, data restoration is performed on the corresponding normalized data so that the data more conforming to real distribution can be obtained when data prediction or data generalization is processed on the physiological feature data of the target object under the at least one physiological indicator based on the target network model. This technical scheme solves the problem in the related art that simulated physiological feature data does not conform to the distribution of real physiological feature data in the simulation of physiological feature data, thereby achieving the effect of obtaining sufficient physiological feature data samples by performing data generalization based on a small amount of physiological feature data on the premise of conforming to the distribution law of the real physiological feature data.

Embodiment three

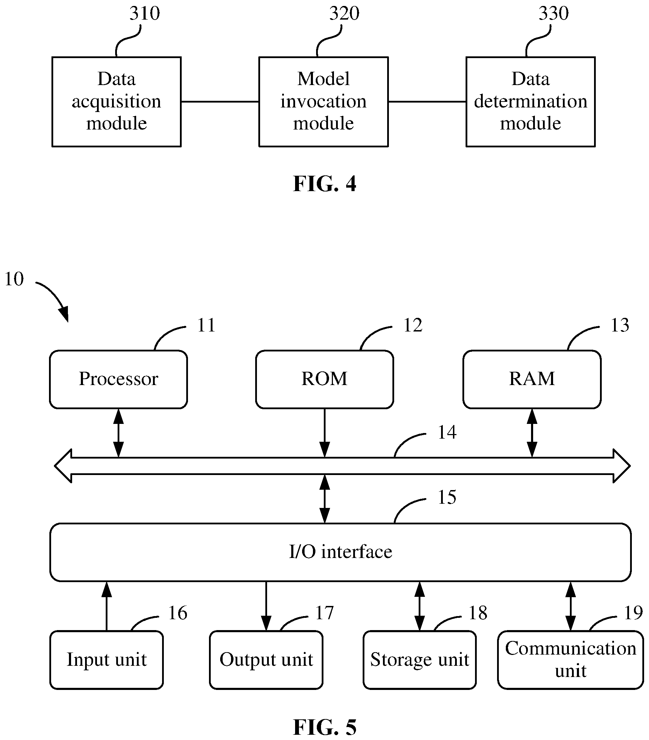

FIG. 4 is a structural diagram of a data processing apparatus according to embodiment three of the present application. As shown in FIG. 4, the apparatus includes a data acquisition module 310, a model invocation module 320, and a data determination module 330.

The data acquisition module 310 is configured to acquire the physiological feature data of a target object under at least one physiological indicator.

The model invocation module 320 is configured to determine a data processing type corresponding to the physiological feature data and invoke a target network model corresponding to the data processing type. The data processing type includes a data generalization type or a data prediction type.

The data determination module 330 is configured to process the physiological feature data based on the target network model to obtain target physiological feature data.

For the technical scheme in the embodiment of the present application, the physiological feature data of the target object under the at least one physiological indicator is acquired. The physiological feature data of the target object is collected; alternatively, the physiological feature data of the target object under the at least one physiological indicator is acquired from a pre-established or existing public data set. The data processing type corresponding to the physiological feature data is determined according to data processing requirements in practical applications, and the target network model corresponding to the data processing type is invoked so that the association relationship between physiological feature data under multiple physiological indicators is analyzed based on the target network model to obtain the target physiological feature data corresponding to the data processing type. The physiological feature data is processed based on the target network model to obtain the target physiological feature data. When data generalization is processed, the physiological feature curve corresponding to the physiological feature data of the target object is determined based on the target network model, and the data generalization is processed based on the physiological feature curve according to the preset data generalization number and data generalization range to obtain at least one target physiological feature curve. When data prediction is processed, physiological feature data of the target object under other physiological indicators may be obtained through the physiological feature data of the target object under the at least one physiological indicator and the basic attribute information of the target object so as to perform the data prediction. This technical scheme solves the problem of insufficient physiological feature data samples when a medicinal property is evaluated based on the physiological feature data or the problem that simulated physiological feature data does not conform to the distribution of real physiological feature data in the simulation of physiological feature data, and the effect of obtaining sufficient physiological feature data samples can be achieved by performing data generalization based on a small amount of physiological feature data on the premise of conforming to the distribution law of real physiological feature data.

Alternatively, the data processing apparatus further includes a matrix construction module, a matrix determination module, and a model determination module. The matrix construction module is configured to, for at least one to-be-processed object, determine the to-be-trained sample data of each to-be-processed object under at least two physiological indicators separately and construct a to-be-processed matrix based on multiple pieces of to-be-trained sample data. Each column in the to-be-processed matrix represents a physiological feature indicator corresponding to the multiple pieces of to-be-trained sample data. Each row in the to-be-processed matrix corresponds to the multiple pieces of to-be-trained sample data.

The matrix determination module is configured to perform a normalization process on each column in the to-be-processed matrix to obtain a to-be-spliced submatrix and perform splicing on the to-be-spliced submatrix to obtain a to-be-used matrix.

The model determination module is configured to input the to-be-used matrix into a to-be-trained network model and train the to-be-trained network model based on the to-be-used matrix until the to-be-trained network model has the minimum loss function to obtain the target network model. The to-be-trained network model includes a generative adversarial network model, a variational autoencoder model, a diffusion model, or a flow-based generation model.

In an embodiment, the data acquisition module includes a first data acquisition unit and a second data acquisition unit. The first data acquisition unit is configured to input the physiological feature data of the target object under the at least one physiological indicator in at least one editing control on a target display interface.

The second data acquisition unit is configured to invoke the physiological feature data of the target object under the at least one physiological indicator from a target database. The target database includes at least one reference object and physiological feature data matching each reference object under the at least one physiological indicator.

In an embodiment, the model invocation module includes a data processing manner determination unit and a model invocation unit. The data processing manner determination unit is configured to receive a data processing instruction and acquire a data processing manner in the data processing instruction. The data processing manner includes a data generalization manner or a data prediction manner.

The model invocation unit is configured to determine the corresponding data processing type based on the data processing manner and the physiological feature data and invoke the target network model corresponding to the data processing type.

In an embodiment, the data determination module includes a physiological feature curve determination unit, a physiological feature generalization curve determination unit, and a first target physiological feature data determination unit. The physiological feature curve determination unit is configured to, when the data processing manner is the data generalization manner, determine a physiological feature curve corresponding to the physiological feature data based on the target network model.

The physiological feature generalization curve determination unit is configured to determine a data floating range corresponding to the physiological feature curve to obtain a preset number of physiological feature generalization curves from the data floating range.

The first target physiological feature data determination unit is configured to obtain at least one group of target physiological feature data corresponding to the physiological feature data based on the physiological feature generalization curves.

In an embodiment, the model invocation module includes a to-be-determined physiological feature curve determination unit, a target physiological feature curve determination unit, and a second target physiological feature data determination unit. The to-be-determined physiological feature curve determination unit is configured to, when the data processing manner is the data prediction manner, determine at least one to-be-determined physiological feature curve matching the physiological feature data based on the target network model.

The target physiological feature curve determination unit is configured to determine a target physiological feature curve from the at least one to-be-determined physiological feature curve according to the basic attribute information corresponding to the target object.

The second target physiological feature data determination unit is configured to determine the target physiological feature data corresponding to the target object based on the target physiological feature curve.

In an embodiment, the physiological feature data includes at least one of height, weight, temperature data, blood pressure data, electrocardiogram data, or biological tissue data.

The data process apparatus according to embodiments of the present application can execute the data process method according to any embodiment of the present application and has functional modules and effects corresponding to the execution methods.

Embodiment four

FIG. 5 is a structural diagram of an electronic device 10 according to an embodiment of the present application. The electronic device is intended to represent various forms of digital computers, for example, a laptop computer, a desktop computer, a worktable, a personal digital assistant, a server, a blade server, a mainframe computer, or another applicable computer. The electronic device may also represent various forms of mobile apparatuses, for example, a personal digital assistant, a cellphone, a smartphone, a wearable device (such as a helmet, glasses, or a watch), or another similar computing apparatus. Herein the shown components, the connections and relationships between these components, and the functions of these components are merely illustrative and are not intended to limit the implementation of the present application as described and/or claimed herein.

As shown in FIG. 5, the electronic device 10 includes at least one processor 11 and a memory, such as a read-only memory (ROM) 12 or a random-access memory (RAM) 13, communicatively connected to the at least one processor 11. The memory stores a computer program executable by the at least one processor. The at least one processor 11 may perform various types of appropriate operations and processing according to a computer program stored in the ROM 12 or a computer program loaded from a storage unit 18 to the RAM 13. The RAM 13 may also store various programs and data required for the operation of the electronic device 10. The at least one processor 11, the ROM 12, and the RAM 13 are connected to each other through a bus 14. An input/output (I/O) interface 15 is also connected to the bus 14.

Multiple components in the electronic device 10 are connected to the I/O interface 15. The multiple components include an input unit 16 such as a keyboard and a mouse, an output unit 17 such as various types of displays and speakers, the storage unit 18 such as a magnetic disk and an optical disk, and a communication unit 19 such as a network card, a modem and a wireless communication transceiver. The communication unit 19 allows the electronic device 10 to exchange information/data with other devices over a computer network such as the Internet and/or various telecommunications networks.

The at least one processor 11 may be various general-purpose and/or special-purpose processing components having processing and computing capabilities. Some examples of a processor 11 include, but are not limited to, a central processing unit (CPU), a graphics processing unit (GPU), a special-purpose artificial intelligence (AI) computing chip, a processor executing machine learning models and algorithms, a digital signal processor (DSP), and any appropriate processor, controller and microcontroller. The processor 11 performs the various methods and processing described above, such as the data processing method.

In some embodiments, the data processing method may be implemented as a computer software program tangibly contained in a computer-readable storage medium such as the storage unit 18. In some embodiments, part or all of computer programs may be loaded and/or installed onto the electronic device 10 via the ROM 12 and/or the communication unit 19. When the computer program is loaded to the RAM 13 and executed by the processor 11, one or more steps of the preceding data processing method may be executed. Alternatively, in other embodiments, the processor 11 may be configured, in any other suitable manner (for example, by means of firmware), to perform the data processing method.

The various embodiments of the systems and techniques described herein may be implemented in digital electronic circuitry, integrated circuitry, a field-programmable gate array (FPGA), an application-specific integrated circuit (ASIC), application specific standard parts (ASSP), a system on a chip (SoC), a complex programmable logic device (CPLD), computer hardware, firmware, software and/or a combination thereof. These embodiments may include implementations in one or more computer programs. The one or more computer programs are executable and/or interpretable on a programmable system including at least one programmable processor. The programmable processor may be a special-purpose or general-purpose programmable processor for receiving data and instructions from a memory system, at least one input device and at least one output device and transmitting data and instructions to the memory system, the at least one input device and the at least one output device.

Computer programs for implementation of the data processing method of the present application may be written in any combination of one or more programming languages. These computer programs may be provided for a processor of a general-purpose computer, a special-purpose computer or another programmable data processing apparatus such that the computer programs, when executed by the processor, cause functions/operations specified in the flowcharts and/or block diagrams to be implemented. The computer programs may be executed entirely on a machine, partly on a machine, as a stand-alone software package, partly on a machine and partly on a remote machine, or entirely on a remote machine or a server.

In the context of the present application, the computer-readable storage medium may be a tangible medium that may contain or store the computer programs that are used by or in conjunction with a system, apparatus or device that executes instructions. The computer-readable storage medium may include, but is not limited to, an electronic, magnetic, optical, electromagnetic, infrared or semiconductor system, apparatus or device, or any suitable combination thereof. Alternatively, the computer-readable storage medium may be a machine-readable signal medium. Examples of the machine-readable storage medium may include an electrical connection based on one or more wires, a portable computer disk, a hard disk, a RAM, a ROM, an erasable programmable read-only memory (EPROM), a flash memory, an optical fiber, a portable compact disk read-only memory (CD-ROM), an optical memory device, a magnetic memory device, or any suitable combination thereof.

To provide interaction with a user, the systems and techniques described herein may be implemented on an electronic device. The electronic device has a display apparatus for displaying information to the user, such as a cathode-ray tube (CRT) or a liquid-crystal display (LCD) monitor, and a keyboard and a pointing apparatus such as a mouse or a trackball through which the user can provide input to the electronic device. Other types of apparatuses may also be used for providing interaction with a user. For example, feedback provided for the user may be sensory feedback in any form (for example, visual feedback, auditory feedback, or haptic feedback). Moreover, input from the user may be received in any form (including acoustic input, voice input, or haptic input).

The systems and techniques described herein may be implemented in a computing system including a back-end component (for example, a data server), a computing system including a middleware component (for example, an application server), a computing system including a front-end component (for example, a client computer having a graphical user interface or a web browser through which a user can interact with implementations of the systems and techniques described herein), or a computing system including any combination of such back-end, middleware or front-end components. Components of a system may be interconnected by any form or medium of digital data communication (for example, a communication network). Examples of the communication network include a local area network (LAN), a wide area network (WAN), a blockchain network, and the Internet.

A computing system may include a client and a server. The client and the server are usually far away from each other and generally interact through the communication network. The relationship between the client and the server arises by virtue of computer programs running on respective computers and having a client-server relationship with each other. The server may be a cloud server, also referred to as a cloud computing server or a cloud host. As a host product in a cloud computing service system, the server solves the defects of difficult management and weak service scalability in a conventional physical host and a virtual private server (VPS).

It is to be understood that various forms of the preceding flows may be used with steps reordered, added, or removed. For example, the steps described in the present application may be executed in parallel, in sequence or in a different order as long as the desired results of the technical schemes in the present application are achieved. The execution sequence of these steps is not limited herein.

The scope of the present application is not limited to the preceding embodiments.

**Claims**

1. A data processing method, comprising:

acquiring physiological feature data of a target object under at least one physiological indicator;

determining a data processing type corresponding to the physiological feature data, and invoking a target network model corresponding to the data processing type, wherein the data processing type comprises a data generalization type or a data prediction type; and

processing the physiological feature data based on the target network model to obtain target physiological feature data.

2. The method according to claim 1, further comprising:

for each of at least one to-be-processed object, determining to-be-trained sample data under at least two physiological indicators, and constructing a to-be-processed matrix based on a plurality of pieces of to-be-trained sample data, wherein each column in the to-be-processed matrix represents a physiological feature indicator corresponding to the plurality of pieces of to-be-trained sample data, and each row in the to-be-processed matrix corresponds to the plurality of pieces of to-be-trained sample data;

performing a normalization process on each column in the to-be-processed matrix to obtain a to-be-spliced submatrix, and splicing the to-be-spliced submatrix to obtain a to-be-used matrix; and

inputting the to-be-used matrix into a to-be-trained network model, and training the to-be-trained network model based on the to-be-used matrix until the to-be-trained network model has a minimum loss function to obtain the target network model, wherein the to-be-trained network model comprises a generative adversarial network model, a variational autoencoder model, a diffusion model, or a flow-based generation model.

3. The method according to claim 1, wherein acquiring the physiological feature data of the target object under the at least one physiological indicator comprises:

inputting the physiological feature data of the target object under the at least one physiological indicator in at least one editing control on a target display interface; or

invoking the physiological feature data of the target object under the at least one physiological indicator from a target database, wherein the target database comprises at least one reference object and physiological feature data matching each of the at least one reference object under the at least one physiological indicator.

4. The method according to claim 1, wherein determining the data processing type corresponding to the physiological feature data, and invoking the target network model corresponding to the data processing type comprises:

receiving a data processing instruction, and acquiring a data processing manner in the data processing instruction, wherein the data processing manner comprises a data generalization manner or a data prediction manner; and

determining the corresponding data processing type based on the data processing manner and the physiological feature data, and invoking the target network model corresponding to the data processing type.

5. The method according to claim 1, wherein the data processing manner is the data generalization manner, and processing the physiological feature data based on the target network model to obtain the target physiological feature data comprises:

determining, based on the target network model, a physiological feature curve corresponding to the physiological feature data;

determining a data floating range corresponding to the physiological feature curve to obtain a preset number of physiological feature generalization curves from the data floating range; and

obtaining, based on the physiological feature generalization curves, at least one group of target physiological feature data corresponding to the physiological feature data.

6. The method according to claim 4, wherein the data processing manner is the data prediction manner, and processing the physiological feature data based on the target network model to obtain the target physiological feature data comprises:

determining, based on the target network model, at least one to-be-determined physiological feature curve matching the physiological feature data;

determining a target physiological feature curve from the at least one to-be-determined physiological feature curve according to basic attribute information corresponding to the target object; and

determining, based on the target physiological feature curve, the target physiological feature data corresponding to the target object.

7. The method according to claim 1, wherein the at least one physiological indicator comprises at least one of height, weight, temperature, blood pressure, electrocardiogram information, or biological tissue information.

8. A data processing apparatus, comprising:

a data acquisition module configured to acquire physiological feature data of a target object under at least one physiological indicator;
a model invocation module configured to determine a data processing type corresponding to the physiological feature data and invoke a target network model corresponding to the data processing type, wherein the data processing type comprises a data generalization type or a data prediction type; and
a data determination module configured to process the physiological feature data based on the target network model to obtain target physiological feature data.

9. An electronic device, comprising:

at least one processor; and
a memory communicatively connected to the at least one processor;
wherein the memory stores a computer program executable by the at least one processor, and the computer program is executed by the at least one processor to cause the at least one processor to perform the data processing method according to any one of claims 1 to 7.

10. A computer-readable storage medium storing computer instructions configured to, when executed, cause a processor to perform the data processing method according to any one of claims 1 to 7.

FIG. 1

S210

For at least one to-be-processed object, determine the to-be-trained sample data of each to-be-processed object under at least two physiological indicators separately, and construct a to-be-processed matrix based on multiple pieces of to-be-trained sample data

S220

Perform a normalization process on each column in the to-be-processed matrix to obtain a to-be-spliced submatrix, and splice the to-be-spliced submatrix to obtain a to-be-used matrix

S230

Input the to-be-used matrix into a to-be-trained network model, and train the to-be-trained network model based on the to-be-used matrix until the to-be-trained network model has the minimum loss function to obtain the target network model

S240

Acquire physiological feature data of the target object under at least one physiological indicator

S250

Determine a data processing type corresponding to the physiological feature data, and invoke the target network model corresponding to the data processing type

S260

Process the physiological feature data based on the target network model to obtain target physiological feature data

**FIG. 2**

Take an acquired human data set as input of a generation model

↓

Data pre-processing

↓

**Training process**

Forward diffusion process: add certain rounds of Gaussian noise to the input data so that the input data set finally converges as the standard Gaussian noise with the same dimension, and optimize the logarithmic likelihood of the distribution generated by the maximization model under the real data distribution to train and obtain a neural network responsible for the forward noise-adding process

↓

**Sampling process**

Reverse diffusion process: Starting with the standard Gaussian noise finally obtained in the forward diffusion process, perform denoising inference and restoration according to multiple rounds of time steps; the probability distribution of each step is obtained from the probability distribution of the forward diffusion process by using the Bayesian formula

↓

As the round number of time steps decreases to 1, the restoration result gradually approaches the real data distribution

↓

Construct the joint distribution of multi-dimensional physiological features

**FIG. 3**

| 310 | 320 | 330 |
|-----|-----|-----|
| Data acquisition module | Model invocation module | Data determination module |

**FIG. 4**

10

| | 11 | | 12 | | 13 |
|---|---|---|---|---|---|
| | Processor | | ROM | | RAM |

14

I/O interface — 15

| 16 | 17 | 18 | 19 |
|----|----|----|----|
| Input unit | Output unit | Storage unit | Communication unit |

**FIG. 5**

## INTERNATIONAL SEARCH REPORT

| International application No. |
| --- |
| **PCT/CN2023/105216** |

**A. CLASSIFICATION OF SUBJECT MATTER**

G16H70/40(2018.01)i

According to International Patent Classification (IPC) or to both national classification and IPC

**B. FIELDS SEARCHED**

Minimum documentation searched (classification system followed by classification symbols)

IPC:G16H,G06F

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 生理指标, 类型, 网络, 模型, 训练, physiological feature, type, network, model, train

**C. DOCUMENTS CONSIDERED TO BE RELEVANT**

| Category* | Citation of document, with indication, where appropriate, of the relevant passages | Relevant to claim No. |
| --- | --- | --- |
| PX | CN 115966314 A (POLIXIR (NANJING) TECHNOLOGIES CO., LTD. et al.) 14 April 2023 (2023-04-14)<br>claims 1-10 | 1-10 |
| X | US 2004002634 A1 (NOKIA CORP.) 01 January 2004 (2004-01-01)<br>description, paragraphs [0007]-[0011] and [0024]-[0046] | 1-10 |
| A | CN 115249043 A (JIANGSU BAOWANGDA SOFTWARE TECHNOLOGY CO., LTD.) 28 October 2022 (2022-10-28)<br>entire document | 1-10 |
| A | CN 114386479 A (BEIJING FRIENDSHIP HOSPITAL, CAPITAL MEDICAL UNIVERSITY) 22 April 2022 (2022-04-22)<br>entire document | 1-10 |
| A | US 2018330062 A1 (AMGEN INC.) 15 November 2018 (2018-11-15)<br>entire document | 1-10 |
| A | US 2007038093 A1 (PETRAGLIA, Felice et al.) 15 February 2007 (2007-02-15)<br>entire document | 1-10 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 September 2023** | **15 September 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
**Information on patent family members**

International application No.

**PCT/CN2023/105216**

| Patent document cited in search report | | | Publication date (day/month/year) | Patent family member(s) | | | Publication date (day/month/year) |
|---|---|---|---|---|---|---|---|
| CN | 115966314 | A | 14 April 2023 | None | | | |
| US | 2004002634 | A1 | 01 January 2004 | US | 2005101845 | A1 | 12 May 2005 |
| CN | 115249043 | A | 28 October 2022 | None | | | |
| CN | 114386479 | A | 22 April 2022 | None | | | |
| US | 2018330062 | A1 | 15 November 2018 | CA | 3072485 | A1 | 14 February 2019 |
| | | | | WO | 2019032391 | A1 | 14 February 2019 |
| | | | | AU | 2018313712 | A1 | 27 February 2020 |
| | | | | EP | 3665706 | A1 | 17 June 2020 |
| US | 2007038093 | A1 | 15 February 2007 | EP | 1743581 | A1 | 17 January 2007 |

Form PCT/ISA/210 (patent family annex) (July 2022)

**REFERENCES CITED IN THE DESCRIPTION**

**Patent documents cited in the description**

- CN 202211667413